# EUROPÄISCHE PATENTANMELDUNG

(11) **EP 3 093 030 A1**
(43) Veröffentlichungstag der Anmeldung: **16.11.2016**
(21) Anmeldenummer: 16165309.2
(22) Anmeldetag: 14.04.2016
(51) Int. Cl.: A61L 27/44, A61L 27/48, C08L 67/04

(54) **BIORESORBIERBARES OSTEOSYNTHESE-IMPLANTAT**

(30) Priorität: 12.05.2015 US 201562159968 P
(71) Anmelder: Biotronik AG, 8180 Bülach (CH)
(72) Erfinder: Rothstock, Stephan, 18057 Rostock (DE); Forkmann, Christoph, 18057 Rostock (DE)
(74) Vertreter: Galander, Marcus

(57) **Zusammenfassung**

Bioresorbierbares Osteosynthese-Implantat, mit einem polymeren Grundkörper und mindestens einer in den Grundkörper integrierten makroskopischen Verstärkungsstruktur aus einem biodegradierbaren Metall oder einer Metalllegierung.

## Beschreibung

Die Erfindung betrifft ein bioresorbierbares Osteosynthese-Implantat mit einem polymeren Grundkörper.

Osteosynthese-Implantate zur Versorgung von Frakturen, Korrektur von Fehlstellungen etc. aus bioverträglichen Metallen, wie Titan oder Titanlegierungen oder rostfreiem Edelstahl, sind seit langem bekannt und massenhaft in Gebrauch. Permanente Implantate aus solchen Materialien verbleiben über den Zeitraum hinaus, in dem sie zur Stabilisierung der Fraktur benötigt werden, im Körper und können zu Komplikationen führen und zusätzliche Eingriffe zur Explantation erfordern. Weiterhin halten sie permanent den Frakturspalt frei von mechanischen Belastungen und verhindern damit eine gewisse mechanische Stimulation, die der Frakturheilung förderlich wäre.

Aufgrund dieser und anderer Nachteile gibt es seit längerer Zeit Bemühungen, optimale resorbierbare Osteosynthese-Implantate zu entwickeln. Als Material für solche resorbierbaren Implantate wurden insbesondere Polymere in Betracht gezogen. Polymere besitzen i.d.R. eine deutlich geringere Steifigkeit als metallische Werkstoffe, wodurch Polymerimplantate größere Querschnitte aufweisen müssen als gleich steife Metallimplantate.

Abbaubare Implantate aus Magnesium werden derzeit erfolgreich für den Einsatz als Kronarstents erprobt. Im Vergleich zu vaskulären Implantaten weisen Osteosynthese-Implantate ein deutlich größeres Volumen auf. Dadurch entstehen während der Degradation problematisch hohe Mengen an Abbauprodukten, vor allem Wasserstoff, die vom Körper nicht schnell genug absorbiert werden können.

Aus der WO 2010/034098 A1 ist ein biodegradierbares medizinisches Implantat bekannt, welches unter anderem als Osteosynthese-Implantat ausgeführt sein kann und aus jeweils feinkörnigem metallischem und polymeren Material gebildet ist. Das metallische Material kann als kleine Teilchen, Fasern oder Flocken in einen Polymerkörper eingebettet sein.

Die US 2011/0054629 A1 beschreibt ein Komposit-Implantat, welches eine poröse Struktur aufweist, die mit einer biodegradierbaren Legierung oder Magnesium mikroskopisch gefüllt ist. Die poröse Grundstruktur kann aus einem Metall, einer Keramik oder einem Polymeren hergestellt sein.

Der Erfindung liegt die Aufgabe zu Grunde, ein verbessertes resorbierbares Osteosynthese-Implantat bereitzustellen, welches insbesondere Abbauprodukte nicht schneller entstehen lässt als sie vom Körper aufgenommen werden können, ausreichende Steifigkeit bei möglichst geringen Dimensionen aufweist und im Verlauf der Frakturheilung mehr und mehr Last auf den heilenden Knochen überträgt.

Diese Aufgabe wird durch ein bioresorbierbares Osteosynthese-Implantat mit den Merkmalen des Anspruchs 1 gelöst. Zweckmäßige Fortbildungen des Erfindungsgedankens sind Gegenstand der abhängigen Ansprüche.

Bei einem solchen Implantat ist in einen polymeren Grundkörper mindestens eine makroskopische Verstärkungsstruktur aus einem biodegradierbaren Metall oder einer entsprechenden Metalllegierung integriert. Mit dem Begriff "integriert" sollen auch Konstruktionen erfasst werden, bei denen die Verstärkungsstruktur partiell aus dem polymeren Grundkörper herausragt.

Durch geeignete Anordnung der Verstärkungsstruktur können Zug-, Biege- und Torsionssteifigkeit des Implantats unabhängig voneinander beeinflusst werden. Auch können Steifigkeit, Degradationszeit und zeitlicher Verlauf der Steifigkeit während der Degradation in verschiedenen Bereichen des Implantats unterschiedlich eingestellt werden. Das Implantat kann vollständig bioresorbierbar gestaltet werden. Die Entwicklung von Wasserstoff bei der Magnesiumdegradation ist deutlich geringer als bei einem vollständig aus Magnesium bestehenden Implantat. Trotzdem kann die im Vergleich zu Polymeren höhere Steifigkeit von Magnesium genutzt werden.

In einer Ausführung der Erfindung umfasst die Verstärkungsstruktur eine Mehrzahl von Verstärkungsstreben in mindestens einem Bereich des Osteosynthese-Implantats. Es kann sich hierbei insbesondere um einen mehr oder weniger zentralen Bereich des Implantats handeln, in dem diesem bei bestimmungsgemäßem Einsatz einer oder mehrere Frakturspalte einer zu versorgenden Knochenstruktur gegenüberliegen. In aus derzeitiger Sicht zweckmäßigen Ausführungen weisen die Verstärkungsstreben insbesondere eine Breite/Dicke im Bereich zwischen 10 um und 5 mm, spezieller zwischen 50 um und 2 mm und noch spezieller zwischen 100 µm und 1 mm, auf.

Anstelle von Verstärkungsstreben kann auch ein kompakter Körper aus dem biodegradierbaren Metall oder der Metalllegierung eingebettet sein, oder es kann von einem solchen kompakten Körper eine Mehrzahl von in den umgebenden polymeren Grundkörper hineinragenden Verstärkungsstreben ausgehen.

In einer weiteren Ausführung der Erfindung ist das vorgeschlagene Implantat als Leiste oder langgestreckte Platte ausgebildet und hat parallel zu deren Längskanten verlaufende Verstärkungsstreben. In aus derzeitiger Sicht zweckmäßigen Ausführungen hat die Leiste bzw. Platte eine Breite/Dicke im Bereich zwischen 200 µm und 20 mm, spezieller zwischen 500 µm und 10 mm und noch spezieller zwischen 1 und 5 mm.

Hierbei ist in einer Ausgestaltung vorgesehen, dass mindestens ein Teil der parallel zu den Längskanten der Leiste oder Platte verlaufenden Verstärkungsstreben sich über die gesamte Länge der Leiste oder Platte erstreckt. Zusätzlich oder auch alternativ hierzu können Verstärkungsstreben vorgesehen sein, die sich nur über einen Teil der Länge des Implantats erstrecken, insbesondere über den weiter oben erwähnten zentralen Bereich, dem im Gebrauch des Implantats einer oder mehrerer Frakturspalte gegenüber liegen.

In einer anderen Ausführung der Erfindung ist das vorgeschlagene Implantat ebenfalls als Leiste oder langgestreckte Platte ausgebildet, hat aber mindestens eine erste und zweite Gruppe von geneigt zu den Längskanten der Leiste oder Platte verlaufenden Verstärkungsstreben, wobei die Verstärkungsstreben der ersten und zweiten Gruppe sich in einem Bereich des Osteosynthese-Implantats überkreuzen. In einer Ausgestaltung dieser Ausführung weist das Implantat mehrere erste und zweite Gruppen von Verstärkungsstreben auf, die sich in jeweils einem Überkreuzungsbereich überkreuzen.

In einer weiteren Ausführung sind zwei vorgenannte Varianten kombiniert, indem geneigt zu den Längskanten der Leiste oder Platte verlaufende Verstärkungsstreben zwischen längs der gegenüberliegenden Kanten verlaufenden parallelen Verstärkungsstreben, diese funktionsmäßig miteinander verbindend, vorgesehen sind. Die geneigt verlaufenden Verstärkungsstreben können dabei als ein Teil des Längsverlaufes der kantenparallelen Verstärkungsstreben ausgeführt sein, sie können aber auch in einer anderen Höhenebene des polymeren Grundkörpers als separate Funktionselemente vorgesehen sein.

In einer Variante der erwähnten Kombination ist die Verstärkungsstruktur als Gitterstruktur von parallel zu den Längskanten der Leiste oder Platte verlaufenden und senkrecht hierzu verlaufenden Verstärkungsstreben ausgebildet. In einer Modifikation ist die Gitterstruktur nicht aus senkrecht, sondern aus schräg zueinander ausgerichteten Verstärkungsstreben ausgebildet und hat beispielsweise dreieckige oder parallelogrammförmige Verstärkungsstruktur-Zellen.

Für andere Anwendungen ist das vorgeschlagene Implantat ausgebildet als Knochenschraube, mit mindestens einer im Wesentlichen parallel zur Schraubenachse oder um diese gewendelt verlaufenden Verstärkungsstrebe, bevorzugt mehreren Verstärkungsstreben. Die Knochenschraube kann, insbesondere mit einem Durchmesser zwischen 1 mm und 15 mm, spezieller zwischen 2 mm und 10 mm, ausgebildet sein.

Für weitere Applikationen ist das vorgeschlagene Implantat ein Marknagel, mit mindestens einer im Wesentlichen parallel zur Nagelachse verlaufenden Verstärkungsstrebe, bevorzugt mehreren Verstärkungsstreben. Der Marknagel kann aus derzeitiger Sicht insbesondere mit einem Durchmesser zwischen 500 µm und 10 mm und spezieller zwischen 1 mm und 5 mm ausgebildet sein.

In einer weiteren Ausführung ist die Verstärkungsstruktur aus Magnesium oder einer Magnesiumlegierung gebildet. Besonders geeignet sind hier Seltene Erden enthaltende Magnesiumlegierungen, wie beispielsweise WE43.

In weiteren materialseitigen Ausgestaltungen weist der polymere Grundkörper ein Biopolymer auf, wie etwa Poly-L-Lactat, Polyglycolsäure, Copolymere dieser oder ähnliches, und ist insbesondere extrudiert, spritzgegossen oder auch faserverpresst. Es können aber auch Polymere wie Polyethylen eingesetzt werden. Je nach konkretem Einsatzzweck und entsprechenden mechanischen Anforderungen kann der Polymeranteil im Bereich zwischen 50 % und 99 %, spezieller zwischen 60 % und 95 % und noch spezieller zwischen 75 % und 90 %, der Gesamtmasse des Grundkörpers liegen.

In weiteren Ausführungen weist das vorgeschlagene Osteosynthese-Implantat zusätzlich zu dem bioresorbierbaren Grundkörper mit der integrierten Verstärkungsstruktur eine nicht bioresorbierbare Komponente auf. Diese kann ebenfalls in den Grundkörper integriert sein und gegebenenfalls partiell aus diesem hervorstehen. Aus derzeitiger Sicht vorteilhaft erscheinen Ausführungen, bei denen die nicht bioresorbierbare Komponente als Endabschnitt oder Strukturelement eines Endabschnitts einer Leiste oder Platte oder einer Knochenschraube oder eines Marknagels ausgebildet ist. Als spezielle Variante kann hierbei auch vorgesehen sein, dass der bioresorbierbare zentrale Teil eines solchen Verbund-Implantats ausschließlich aus einem biodegradierbaren Metall (ohne Polymeranteil) besteht.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1: eine schematische Seitenansicht einer Ausführungsform der Erfindung in einer Anwendungssituation,
- Fig. 2: eine perspektivische Darstellung eines weiteren Ausführungsbeispiels,
- Fig. 3: eine perspektivische Darstellung eines weiteren Ausführungsbeispiels,
- Fig. 4: eine perspektivische Darstellung eines weiteren Ausführungsbeispiels,
- Fig. 5: eine perspektivische Darstellung eines weiteren Ausführungsbeispiels,
- Fig. 6: eine perspektivische Darstellung einer Knochenschraube in einer Ausführungsform der Erfindung und
- Fig. 7: eine perspektivische Darstellung eines Marknagels gemäß einer Ausführungsform der Erfindung.

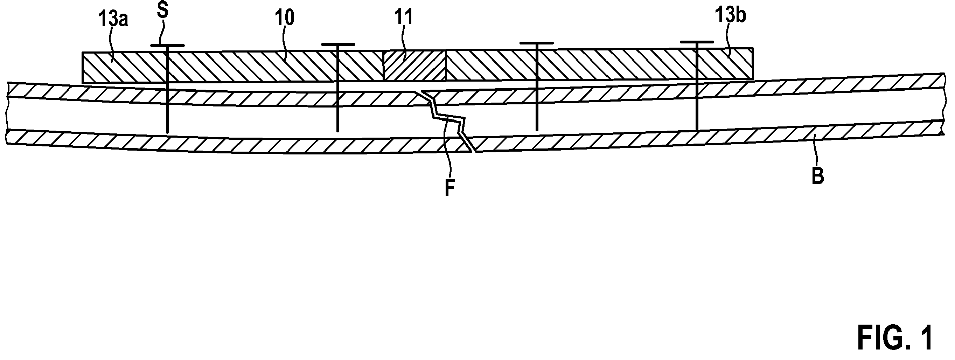

Fig. 1 zeigt schematisch einen frakturierten Knochen B mit Frakturspalt F, an dem mittels mehrerer Knochenschrauben S ein plattenartiges Osteosynthese-Implantat 10 befestigt ist, welches ein als massiver Magnesiumkörper gebildetes, über dem Frakturspalt F positioniertes Mittelteil 11 und zwei aus einem Biopolymeren gebildeten Endabschnitte 13a, 13b umfasst. Das Magnesium-Mittelteil 11 ist im Körper schneller abbaubar als die aus dem Biopolymeren bestehenden Endabschnitte 13a, 13b, wodurch eine sinnvoll gesteuerte Lastübertragung auf den heilenden Knochen B im Verlauf der Frakturheilung ermöglicht wird. Die Endabschnitte 13a, 13b können auch aus einem nicht bioresorbierbaren Material bestehen, etwa einer Titanlegierung.

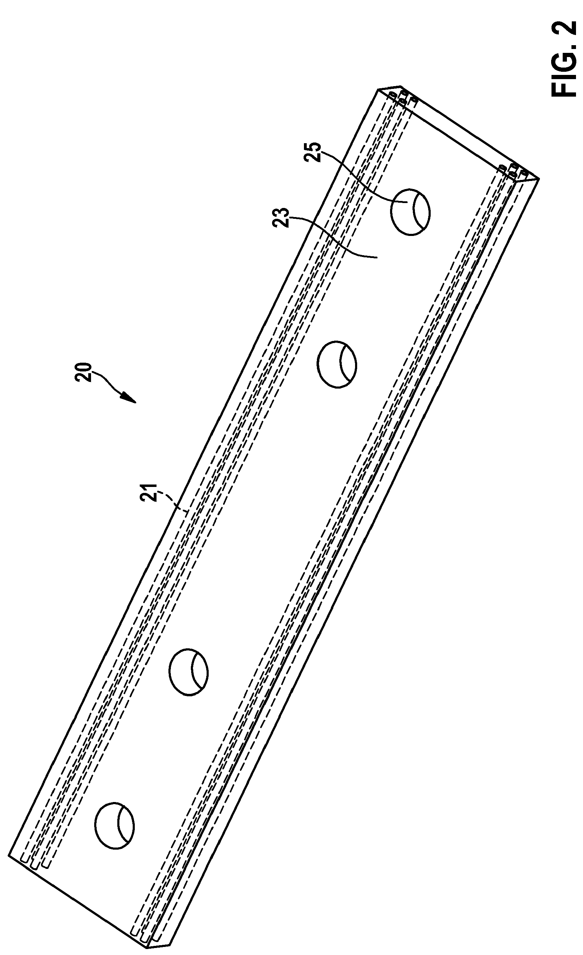

Fig. 2 zeigt in einer perspektivischen Ansicht eine Osteosynthese-Platte 20 mit parallel zu den Längskanten verlaufenden Verstärkungsstreben 21 (etwa aus Mg oder einen Mg-Legierung), eingebettet in einen polymeren Grundkörper 23 (etwa aus Poly-L-Lactat). In dem Grundkörper 23 sind mehrere zylindrische Durchgangslöcher 25 zum Hindurchführen von Knochenschrauben vorgesehen. Die Verstärkungsstreben 21 sind jeweils in einer Gruppe von mehreren Streben von einem zum anderen Ende der Osteosynthese-Platte 20 durchlaufend integriert. Sie dienen zur Erhöhung der Biegesteifigkeit des Implantats über dessen gesamte Länge.

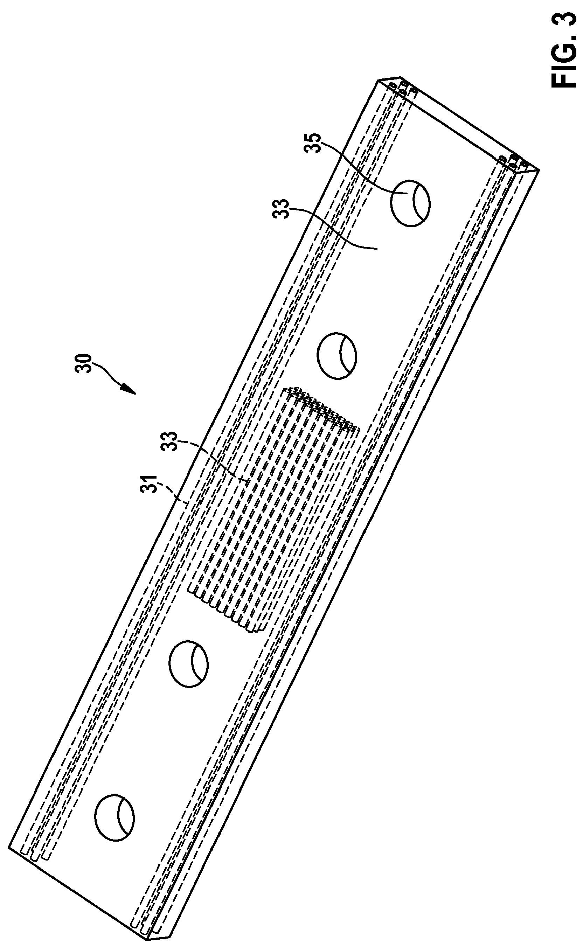

Fig. 3 zeigt als Modifikation der vorab erwähnten Ausführungsform eine weitere Osteosynthese-Platte 30, die neben durchgehenden Gruppen von Verstärkungsstreben 31, die parallel zu den Längskanten der Platte verlaufen, in einem Grundkörper 33 eine zusätzliche zentrale Verstärkungsstruktur 32 hat, die ebenfalls aus dem Material der Verstärkungsstreben 31 gebildet ist. Es kann sich hierbei wiederum um eine Gruppe einzelner Streben, aber auch um ein kammartiges massives Verstärkungsteil handeln. Diese Komponente 32 erhöht zusätzlich die Biegesteifigkeit der Platte 30 in deren Mittenbereich.

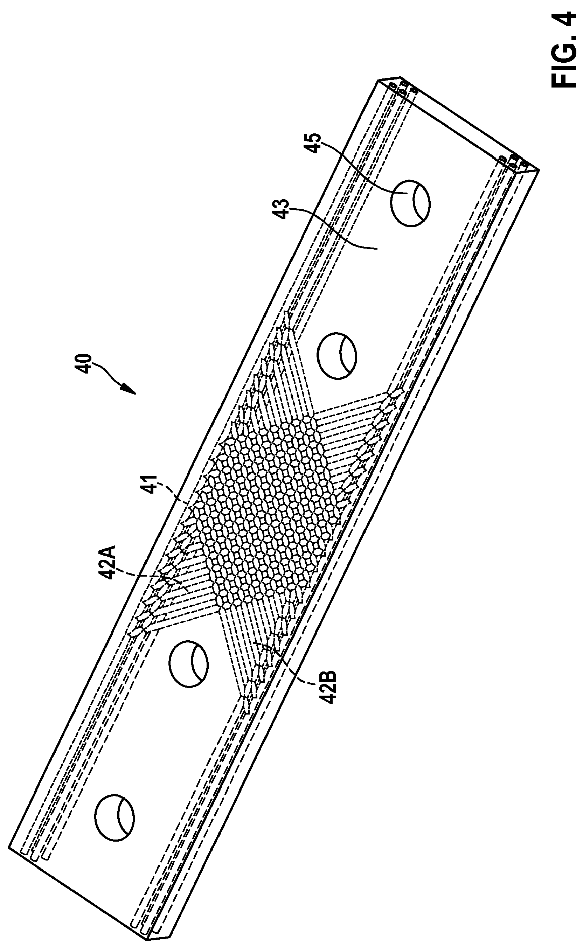

Fig. 4 zeigt, als weitere Abwandlung der Osteosynthese-Platte aus Fig. 2, eine weitere Osteosynthese-Platte 40 mit zwei Gruppen von längs der Längskanten verlaufenden Verstärkungsstreben 41. Hier ist eine zusätzliche Erhöhung der Biegesteifigkeit im mittleren Bereich durch eine erste und zweite Gruppe 42A, 42B von schräg zu den Längsachsen verlaufenden und sich kreuzenden Verstärkungsstreben realisiert. Die Verstärkungsstreben-Gruppen 42A, 42B liegen auf unterschiedlichem Höhenniveau eingebettet in den polymeren Grundkörper 43.

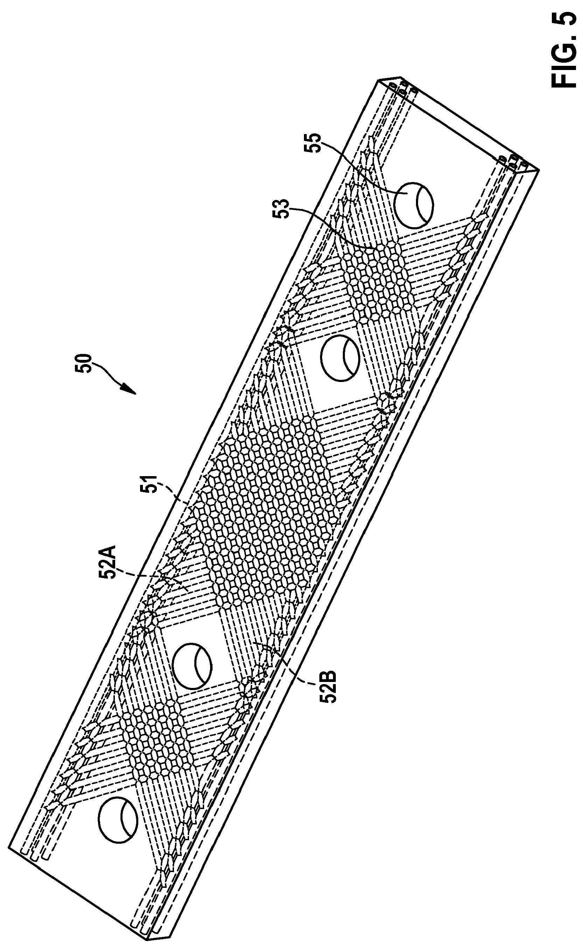

Fig. 5 zeigt, als Abwandlung der vorgenannten Ausführung, eine Osteosynthese-Platte 50, in die jeweils mehrere erste und zweite Gruppen 52A, 52B von schräg zu den Längskanten verlaufenden und sich im Mittenbereich des polymeren Grundkörpers 53 kreuzenden Verstärkungsstreben angeordnet sind. Entsprechend der Mehrzahl der zusätzlichen Verstärkungsstreben-Gruppen wird eine zusätzliche Erhöhung der Biegesteifigkeit nicht nur im Mittenbereich des Implantats, sondern praktisch über dessen gesamte Länge erreicht.

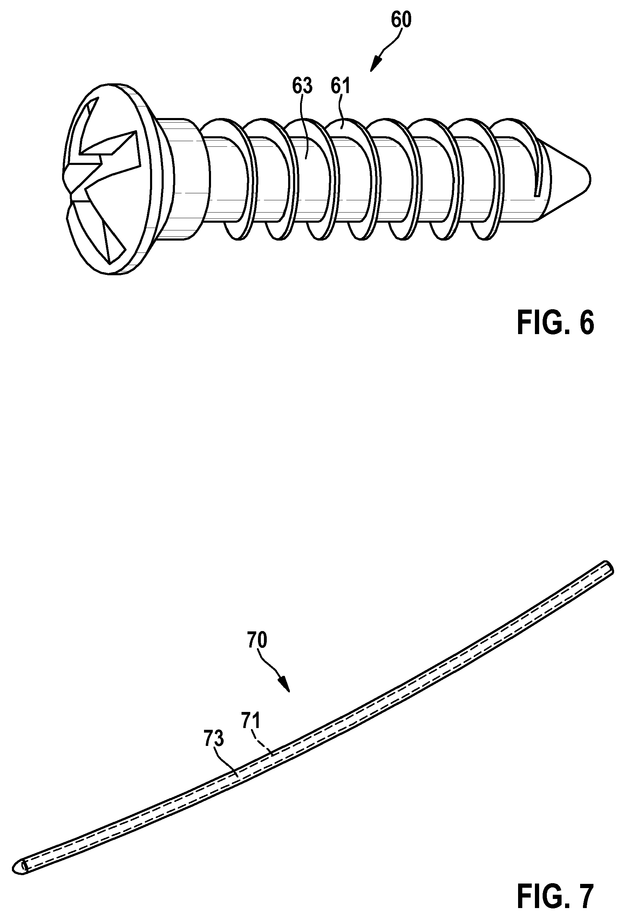

Fig. 6 zeigt eine Knochenschraube 60 mit einem Polymer-Schaubenkörper 63, in dessen Außenumfang als Schraubengang eine Verstärkungswendel 61 aus einer biodegradierbaren Metalllegierung eingefügt ist. Diese Verstärkungsstruktur 61 ist also teilweise in den Grundkörper 63 eingebunden und steht teilweise aus diesem heraus.

Fig. 7 zeigt, als weiteres Ausführungsbeispiel der Erfindung, einen Marknagel 70, bei dem in einen polymeren Grundkörper 73 zentrisch eine (in der Figur gestrichelt gezeichnete) einzelne Verstärkungsstrebe 71 eingebettet ist.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Bioresorbierbares Osteosynthese-Implantat (10; 20; 30; 40; 50; 60; 70), mit einem polymeren Grundkörper (13a, 13b; 23; 33; 43; 53; 63; 73) und mindestens einer in den Grundkörper integrierten makroskopischen Verstärkungsstruktur (11; 21; 31, 32; 41, 42A, 42B; 51; 52A, 52B; 61; 71) aus einem biodegradierbaren Metall oder einer biodegradierbaren Metalllegierung.

2. Osteosynthese-Implantat nach Anspruch 1, wobei die Verstärkungsstruktur eine Mehrzahl von Verstärkungsstreben (21; 31, 32; 41; 42A, 42B; 51, 52A, 52B) in mindestens einem Bereich des Osteosynthese-Implantats (20; 30; 40; 50) umfasst, die insbesondere eine Breite/Dicke im Bereich zwischen 10 µm und 5 mm, spezieller zwischen 50 µm und 2 mm und noch spezieller zwischen 100 µm und 1 mm, aufweisen.

3. Osteosynthese-Implantat nach Anspruch 2, welches als Leiste oder langgestreckte Platte (10; 20; 30; 40; 50), insbesondere mit einer Breite/Dicke im Bereich zwischen 200 µm und 20 mm, spezieller zwischen 500 µm und 10 mm und noch spezieller zwischen 1 und 5 mm, ausgebildet ist und parallel zu den Längskanten der Leiste oder Platte verlaufende Verstärkungsstreben (21; 31; 32; 41; 51) aufweist.

4. Osteosynthese-Implantat nach Anspruch 3, wobei mindestens ein Teil der parallel zu den Längskanten der Leiste oder Platte (10; 20; 30; 40; 50) verlaufenden Verstärkungsstreben (21; 31; 32; 41; 51) sich über die gesamte Länge der Leiste oder Platte erstreckt.

5. Osteosynthese-Implantat nach einem der Ansprüche 2 bis 4, welches als Leiste oder langgestreckte Platte (40; 50), insbesondere mit einer Breite/Dicke im Bereich zwischen 200 µm und 20 mm, spezieller zwischen 500 µm und 10 mm und noch spezieller zwischen 1 und 5 mm, ausgebildet ist und mindestens eine erste und zweite Gruppe von geneigt zu den Längskanten der Leiste oder Platte verlaufenden Verstärkungsstreben (42A, 42B; 52A, 52B) aufweist, wobei die Verstärkungsstreben der ersten und zweiten Gruppe sich in einem zentralen Bereich der Leiste oder Platte überkreuzen.

6. Osteosynthese-Implantat (50) nach Anspruch 5, welches mehrere erste und zweite Gruppen von Verstärkungsstreben (52A; 52B) aufweist, die sich in jeweils einem Überkreuzungsbereich überkreuzen.

7. Osteosynthese-Implantat (40; 50) nach Anspruch 5 oder 6, wobei die geneigt zu den Längskanten der Leiste oder Platte verlaufende Verstärkungsstreben (42A, 42B; 52A, 52B) zwischen längs der gegenüberliegenden Kanten verlaufenden parallelen Verstärkungsstreben (41; 51), diese funktionsmäßig miteinander verbindend, vorgesehen sind.

8. Osteosynthese-Implantat nach einem der Ansprüche 4 bis 7, wobei die Verstärkungsstruktur als Gitterstruktur von parallel zu den Längskanten der Leiste oder Platte verlaufenden und senkrecht hierzu verlaufenden Verstärkungsstreben ausgebildet ist.

9. Osteosynthese-Implantat nach Anspruch 1 oder 2, ausgebildet als Knochenschraube (60), insbesondere mit einem Durchmesser zwischen 1 mm und 15 mm, spezieller zwischen 2 mm und 10 mm, mit mindestens einer im Wesentlichen parallel zur Schraubenachse oder um diese gewendelt verlaufenden Verstärkungsstrebe (61), bevorzugt mehreren Verstärkungsstreben.

10. Osteosynthese-Implantat nach Anspruch 1 oder 2, ausgebildet als Marknagel (70), insbesondere mit einem Durchmesser zwischen 500 µm und 10 mm und spezieller zwischen 1 mm und 5 mm, mit mindestens einer im Wesentlichen parallel zur Nagelachse verlaufenden Verstärkungsstrebe (71), bevorzugt mehreren Verstärkungsstreben.

11. Osteosynthese-Implantat nach einem der vorangehenden Ansprüche, wobei die Verstärkungsstruktur (11; 21; 31, 32; 41, 42A, 42B; 51; 52A, 52B; 61; 71) aus Magnesium oder einer Magnesiumlegierung gebildet ist.

12. Osteosynthese-Implantat nach einem der vorangehenden Ansprüche, wobei der polymere Grundkörper (13a, 13b; 23; 33; 43; 53; 63; 73) ein Biopolymer aufweist, wie etwa Poly-L-Lactat, Polyglycolsäure, Copolymere dieser oder ähnliches, und insbesondere extrudiert, spritzgegossen oder faserverpresst ist.

13. Osteosynthese-Implantat nach einem der vorangehenden Ansprüche, wobei der Polymeranteil im Bereich zwischen 50 % und 99 %, spezieller zwischen 60 % und 95 % und noch spezieller zwischen 75 % und 90 %, der Gesamtmasse des Grundkörpers liegt.

14. Osteosynthese-Implantat (10) nach einem der vorangehenden Ansprüche, welches eine nicht bioresorbierbare Komponente (13a, 13b) aufweist.

15. Osteosynthese-Implantat nach Anspruch 13, wobei die nicht bioresorbierbare Komponente als Endabschnitt (13a, 13b) oder Strukturelement eines Endabschnitts einer Leiste oder Platte (10) oder einer Knochenschraube oder eines Marknagels ausgebildet ist.
